# EUROPÄISCHE PATENTSCHRIFT

(11) **EP 1 742 688 B1**
(45) Veröffentlichungstag und Bekanntmachung des Hinweises auf die Patenterteilung: **19.12.2007**
(21) Anmeldenummer: 05729342.5
(22) Anmeldetag: 19.04.2005
(51) Int. Cl.: A61M 5/315

(54) **INJEKTIONSGERÄT MIT MEHRFACH VERDREHSICHERBARER DOSIEREINRICHTUNG**
INJECTION DEVICE COMPRISING A DOSING UNIT WITH MULTIPLE ANTI-TWIST PROTECTION
APPAREIL D'INJECTION COMPORTANT UN DISPOSITIF DE DOSAGE POUVANT ETRE BLOQUE PLUSIEURS FOIS EN ROTATION

(30) Priorität: 23.04.2004 DE 202004006611 U
(43) Veröffentlichungstag der Anmeldung: 17.01.2007
(73) Patentinhaber: TecPharma Licensing AG, 3401 Burgdorf (CH)
(72) Erfinder: BURREN, Stefan, CH-3047 Bremgarten (CH); MOSER, Ulrich, CH-3412 Heimiswil (CH); SCHRUL, Christian, CH-3427 Utzenstorf (CH); SOMMER, Christoph, CH-3435 Ramsei (CH)
(86) Internationale Anmeldenummer: PCT/CH2005/000217
(87) Internationale Veröffentlichungsnummer: WO 2005/102421

(56) Entgegenhaltungen:
- EP-A- 0 611 035
- WO-A-97/36625
- WO-A-03/033363
- WO-A-20/04030730
- US-A1- 2001 009 990
- US-A1- 2003 158 523

## Beschreibung

Die Erfindung betrifft eine mehrfach verdrehsicherbare Dosiervorrichtung und eine Vorrichtung zur Verabreichung eines injizierbaren Produkts, die eine freie Dosisauswahl eines Verwenders der Vorrichtung erlaubt. Das Injektionsgerät ist insbesondere für solche Verwendungen geeignet, in denen der Verwender sich das Produkt selbst verabreicht und die Dosis pro Verabreichung selbst individuell auswählen, d. h. einstellen, kann. Besonders bevorzugt handelt es sich um ein Injektionsgerät für beispielsweise die Verabreichung von Insulin in der Diabetestherapie oder die Verabreichung eines Wachstumshormons.

Aus der EP 0 713 403 A1 ist eine Spritze für die Verabreichung von flüssigen pharmazeutischen Mischungen und generell auch anderen Flüssigkeiten bekannt, die eine Einstellung einer pro Injektion zu verabreichenden Flüssigkeitsdosis einmalig erlaubt; genannt ist eine Einstellung durch einen Apotheker. Für den Patienten, der sich anschließend mit der Spritze die pharmazeutische Flüssigkeit verabreicht, ist es jedoch schwierig, die einmal eingestellte Dosis zu verändern. Hierdurch soll eine fehlerhafte Verwendung der Spritze verhindert werden. Eine derartige Spritze wird den Bedürfnissen nicht gerecht, da in vielen Therapien die optimale Produktdosis variiert, beispielsweise in Abhängigkeit von der Tageszeit, sportlichen Aktivitäten oder der Einnahme von Mahlzeiten.

Injektionsgeräte, die derartigen Anforderungen gerecht werden, sind beispielsweise aus der WO 97/36625 und DE 199 00 792 C2 bekannt. Die beiden Druckschriften betreffen Injektionsgeräte mit je einer Fördereinrichtung für die Ausschüttung des Produkts und einer Dosiereinrichtung für die Einstellung der Produktdosis, die bei einer nachfolgenden Injektion mittels der Fördereinrichtung förderbar und dadurch ausschüttbar ist. Die Fördereinrichtung umfasst einen Kolben, durch dessen Vorschub das Produkt aus einem Produktreservoir gefördert wird, eine Kolbenstange und ein Antriebsglied für die Kolbenstange. Das Antriebsglied und die Kolbenstange sind miteinander in solch einem Eingriff, dass eine Vorschubbewegung des Antriebsglieds eine ebensolche Vorschubbewegung der Kolbenstange bewirkt, das Antriebsglied jedoch eine Rückzugsbewegung in die Gegenrichtung bis in eine Auslöseposition ausführt, aus der heraus erneut eine Injektion vorgenommen werden kann. Die Auslöseposition wird mittels der Dosiereinrichtung bestimmt, die einen einstellbaren Dosieranschlag für das Antriebsglied bildet. Die bekannten Vorrichtungen haben sich in der Praxis bewährt, sollten die Gefahr von Fehldosierungen aber noch sicherer ausschließen.

Die EP 0 879 610 B1 zeigt eine wiederverwendbare Abgabevorrichtung für Medikamente. Ein an einem Gehäuse der Vorrichtung angebrachter Vorsprung greift in eine von mehreren dem Vorsprung angepassten Nuten ein, die an einem Auswahlelement angebracht wird. Der Vorsprung ist federnd an dem Gehäuse befestigt, wodurch bei einer Verdrehung des Gehäuses gegen das Auswahlelement das Auswahlelement von einer Nut in eine benachbarte Nut springt und somit ein hörbares Klicken erzeugt.

Es ist eine Aufgabe der Erfindung, ein Antriebs- und Dosiermodul und ein Injektionsgerät zur Verabreichung eines injizierbaren Produkts zu schaffen, die eine freie Dosisauswahl bei verringerter Gefahr einer Fehldosierung erlauben.

Die Erfindung betrifft ein Injektionsgerät zur Verabreichung eines injizierbaren Produkts, vorzugsweise ein handliches, in einer Tasche der Kleidung tragbares Injektionsgerät. Das Injektionsgerät umfasst ein Gehäuse mit einem Reservoir für das Produkt, eine Fördereinrichtung für die Förderung des Produkts und eine Dosiereinrichtung für die pro Injektion wahlfrei einstellbare Produktdosis. Das Reservoir kann unmittelbar von dem Gehäuse selbst gebildet werden. Vorzugsweise bildet das Gehäuse allerdings ein Aufnahmefach für ein Produktbehältnis, das vorzugsweise wie üblich als vorabgefüllte Ampulle in den Handel gelangt. Als "Gehäuse mit einem Reservoir" wird auch solch ein Gehäuse verstanden, das ein Aufnahmefach für ein Produktbehältnis bildet, in dem das Produktbehältnis noch nicht eingesetzt ist.

Insbesondere betrifft die Erfindung ein Antriebs- und Dosiermodul eines Injektionsgeräts oder für ein Injektionsgerät.

Die Fördereinrichtung wird von einem Mechanikhalter, den vorzugsweise ein Gehäuseabschnitt bildet, bewegbar gelagert. Sie kann eine Förderbewegung ausführen, durch die das Produkt aus dem Reservoir gefördert und ausgeschüttet wird. Sie kann relativ zu dem Gehäuse oder wenigstens einem Teil des Gehäuses in wenigstens, vorzugsweise genau zwei unterschiedliche Positionen bewegt werden, die vorgegeben sind, vorzugsweise durch Anschläge. Die eine der Positionen ist eine Auslöseposition, aus der heraus die Förderbewegung unmittelbar oder nach vorheriger Ausführung einer anderen Bewegung ausgeführt wird. Die andere der Positionen ist eine Freigabeposition, aus der die Fördereinrichtung in die Auslöseposition bewegbar ist. Die Förderbewegung und die Bewegung in die Auslöseposition sind bevorzugterweise Linearbewegungen, besonders bevorzugt entlang einer einzigen Translationsachse. Die Bewegung aus der Freigabeposition in die Auslöseposition ist der Förderbewegung vorzugsweise exakt entgegengerichtet. Vorzugsweise ist die Fördereinrichtung zwischen der Auslöseposition und der Freigabeposition hin und her bewegbar, besonders bevorzugt ist sie ausschließlich in dieser Art bewegbar. Die Bewegung aus der Auslöseposition wird vorzugsweise durch manuellen Druck auf die Fördereinrichtung und die Bewegung in die Auslöseposition wird vorzugsweise durch eine manuell aufgebrachte Zugkraft bewirkt. Die Bewegung in die Auslöseposition wird im Folgenden vereinfachend als Rücksetzbewegung bezeichnet. Wenn es heißt, dass die Fördereinrichtung eine Bewegung ausführt, so bedeutet dies nicht, dass im Falle einer bevorzugt mehrteiligen Ausführung der Fördereinrichtung sämtliche Teile der Fördereinrichtung die betreffende Bewegung stets oder überhaupt gemeinsam ausführen, obgleich eine gemeinsame Bewegung, zumindest phasenweise, bevorzugt wird.

Die Dosiereinrichtung ist so gelagert, vorzugsweise so mit dem genannten Mechanikhalter verbunden, dass sie relativ zu der Fördereinrichtung oder zumindest einem Teil der Fördereinrichtung eine Dosierbewegung ausführen kann, um die durch die Förderbewegung förderbare Produktdosis einzustellen. Die einstellbare Produktdosis wird durch Dosierpositionen vorgegeben, in die die Dosiereinrichtung bei der Dosierbewegung vorzugsweise verrastet. Der entsprechende Rasteingriff kann mit dem Gehäuse oder/und mit der Fördereinrichtung gebildet sein. Die Dosierung kann vorgenommen werden, wenn die Fördereinrichtung die Freigabeposition einnimmt. Bei den vorgegebenen Dosierpositionen kann es sich um nur zwei unterschiedliche Dosierpositionen handeln, um beispielsweise zu unterschiedlichen Tageszeiten zwei unterschiedliche Produktdosen zu verabreichen. In anderen Ausführungen sind mehr als zwei, vorzugsweise ist eine Vielzahl unterschiedlicher Dosierpositionen vorgesehen, um in Anpassung an unterschiedliche Situationen und/oder einer bezüglich der zu verabreichenden Produktdosen heterogenen Personengruppe die Möglichkeit individueller Dosiseinstellung zu gewähren.

Nach der Erfindung ist die Dosiereinrichtung mit der Fördereinrichtung mittels Blokkiereingriff gekoppelt, wenn die Fördereinrichtung die Auslöseposition einnimmt. Der Blockiereingriff kann auch bereits während der Rücksetzbewegung der Fördereinrichtung bestehen. Im Blockiereingriff ist die Dosiereinrichtung relativ zu der Fördereinrichtung in der zuvor eingestellten Dosierposition gegen Dosierbewegungen blockiert. Eine erzwungene Bewegung aus der blockierten Dosierposition ist allenfalls durch eine außergewöhnliche Kraftanstrengung möglich und hat vorzugsweise eine derart weitgehende Beschädigung der Vorrichtung zur Folge, dass eine nachfolgende Produktverabreichung nicht mehr möglich ist. Vorteil der Erfindung ist, dass die Dosis in der Freigabeposition eingestellt und eine "Nachdosierung" in der Auslöseposition verhindert wird. Durch die Blockierung in der Auslöseposition kann das Injektionsgerät unmittelbar im Zusammenhang mit der Verabreichung sicherer gehandhabt werden, weil die für die Verabreichung erforderlichen Handgriffe nicht versehentlich zu einer Dosierbewegung führen können. Obgleich bevorzugt wird, dass die Dosiereinrichtung im Blokkiereingriff eng geführt wird, um Bewegungen quer zur Richtung der Rücksetzbewegung zu verhindern, kann grundsätzlich ein gewisses Spiel vorhanden sein, solange hierdurch die eingestellte Dosis nicht verstellt werden kann.

Die Auslöseposition wird, wie bereits erwähnt, als Anschlagposition bestimmt. Zwecks Dosierung ist diese Anschlagposition in und gegen die Richtung der Förderbewegung und dadurch die maximale Weglänge der Förderbewegung verstellbar. Die Fördereinrichtung und die Dosiereinrichtung bilden je einen Dosieranschlag, und die beiden Dosieranschläge begrenzen die Rücksetzbewegung der Fördereinrichtung und bestimmen dadurch die Auslöseposition. Dies bedeutet, dass eines aus Fördereinrichtung und Dosiereinrichtung, vorzugsweise die Dosiereinrichtung, einen bezüglich seiner Position veränderbaren Anschlag bildet. Ein Beispiel für die Bildung des verstellbaren Dosieranschlags werden in der WO 97/36625 offenbart, der erfindungsgemäß abgewandelt wird. In kinematischer Umkehr könnten die in dieser Druckschrift offenbarten Dosieranschläge ihrem Verlaufe nach, d. h. als diskrete Dosieranschläge, auch an der Fördereinrichtung gebildet sein, so dass ein Anschlagnocken an der Dosiereinrichtung genügt, der durch die Dosierbewegung in eine Dosierposition eingestellt wird.

In bevorzugten Ausführungen wird die Blockierung der Dosiereinrichtung durch den Eingriff wenigstens eines Auswahlelements zwischen zwei Drehanschläge bewirkt. Sichergestellt wird, dass für jede der Dosierpositionen eines aus Fördereinrichtung und Dosiereinrichtung ein Auswahlelement und das andere zwei zugeordnete Drehanschläge bildet, die miteinander in dem Blockiereingriff sind. Wegen der mehreren unterschiedlichen Dosierpositionen sind entsprechend mehrere Drehanschlagpaare und/oder mehrere Auswahlelemente vorgesehen, von denen wenigstens eine Paarung in jeder der Dosierpositionen in dem Blockiereingriff ist. Vorzugsweise sind mehrere Paarungen in einem Blockiereingriff. Die Drehanschläge oder das Auswahlelement können oder kann an der Fördereinrichtung starr gebildet sein, insbesondere einstückig. Das mindestens eine von der Dosiereinrichtung gebildete Gegenglied kann an der Dosiereinrichtung starr geformt sein, insbesondere einstückig.

Der Eingriff zwischen dem Auswahlelement und den Drehanschlägen kann in der Freigabeposition der Fördereinrichtung gelöst oder als lösbarer Rasteingriff gebildet sein, der vorteilhafterweise gleichzeitig den genannten lösbaren Rasteingriff für die Dosisauswahl in der Freigabeposition der Fördereinrichtung bilden kann. In der zuletzt genannten Variante schwächt sich der Blockiereingriff bei der Bewegung in die Freigabeposition bis auf den Rasteingriff ab; umgekehrt verstärkt sich der in der Freigabeposition bestehende Rasteingriff zu dem Blockiereingriff bei der Rücksetzbewegung der Fördereinrichtung.

Die Drehanschläge können insbesondere durch Führungsnuten oder vorragender Führungsstege gebildet sein, die sich nur über ein kleines Stück oder über nahezu die gesamte Weglänge der Rücksetzbewegung der Fördereinrichtung erstrecken können. Die Drehanschläge können sich im Falle eines starren Auswahlelements so nahe als möglich bis zu dem Auswahlelement erstrecken, wenn die Fördereinrichtung die Freigabeposition einnimmt. Falls das Auswahlelement oder die mehreren Auswahlelemente in der Freigabeposition mit der Führung oder den Drehanschlägen in einem lösbaren Rasteingriff sind, erstrecken sich die Drehanschläge entsprechend über eine verlängerte Strekke.

Die Dosierbewegung umfasst vorzugsweise eine Drehbewegung der Dosiereinrichtung relativ zu der Fördereinrichtung um eine Drehachse. Die Dosierbewegung kann eine reine Drehbewegung sein. Sie kann auch eine überlagerte Dosierbewegung aus Drehbewegung und Translationsbewegung, in diesem Fall vorzugsweise entlang der Drehachse sein. Die Förderbewegung der Fördereinrichtung umfasst vorzugsweise eine Bewegung der Fördereinrichtung relativ zu der Dosiereinrichtung entlang der Drehachse. Die Förderbewegung kann insbesondere eine reine Linearbewegung entlang der Drehachse sein. Insbesondere in solchen Ausbildungen bietet es sich an, dass eine der Strukturen, nämlich die Fördereinrichtung oder die Dosiereinrichtung, das andere um die Drehachse wenigstens teilweise umgibt und die in der erforderlichen Anzahl vorhandenen Drehanschläge und/oder Auswahlelemente an einander zugewandt gegenüberliegenden Mantelflächen der Fördereinrichtung und der Dosiereinrichtung angeordnet sind. Diejenige der beiden Strukturen, vorzugsweise die Dosiereinrichtung, die die andere wenigstens teilweise umgibt, ist oder umfasst vorzugsweise einen Hülsenkörper und bildet vorzugsweise die Drehanschläge.

Die Fördereinrichtung kann einteilig sein, ist vorzugsweise jedoch mehrteilig. Sie umfasst in der mehrteiligen Ausbildung ein Förderglied, das die Förderbewegung ausführt und dabei unmittelbar auf das in dem Reservoir befindliche Produkt wirkt, und eine Antriebseinrichtung, die mit dem Förderglied gekoppelt ist, um dessen Förderbewegung zu bewirken. Die Antriebseinrichtung umfasst vorzugsweise ein Abtriebsglied und ein Antriebsglied, die relativ zueinander bewegbar und miteinander so gekoppelt sind, dass eine Antriebsbewegung des Antriebsglieds eine Abtriebsbewegung des Abtriebsglieds bewirkt. Das Abtriebsglied kann mit dem Förderglied steif verbunden sein oder ist mit dem Förderglied so gekoppelt, dass die Abtriebsbewegung des Abtriebsglieds die Förderbewegung bewirkt. Vorzugsweise nimmt das Abtriebsglied bei seiner Abtriebsbewegung das Förderglied einfach mit. Das Antriebsglied ist so gelagert, dass es zum Einen die Antriebsbewegung und zum Anderen eine der Antriebsbewegung entgegengerichtete Bewegung in die Auslöseposition der Fördereinrichtung ausführen kann. Das Antriebsglied und das Abtriebsglied sind vorzugsweise so miteinander gekoppelt, dass das Antriebsglied bei der Antriebsbewegung das Abtriebsglied mitnimmt, während das Antriebsglied die Bewegung in die Gegenrichtung vorzugsweise ohne das Abtriebsglied ausführt. Derartige Antriebseinrichtungen sind insbesondere von Zahnstangenpens bekannt, beispielsweise aus der WO 97/36625 und der DE 199 00 792 C2. Geeignet ist beispielsweise auch eine Antriebseinrichtung, wie sie die DE 199 45 397 C2 beschreibt, bei der das Abtriebsglied glatt und das Antriebsglied mit sich in die glatte Außenfläche des Abtriebsglieds stemmenden Eingriffselementen gebildet sind. Die Bewegungen der Fördereinrichtung und in der mehrteiligen Ausbildung der Glieder der Fördereinrichtung umfassen oder sind vorzugsweise Linearbewegungen entlang einer Translationsachse der Fördereinrichtung.

Falls die Dosiereinrichtung, wie bevorzugt, die Drehanschläge bildet, kann ihr Dosieranschlag oder können ihre vorzugsweise mehreren Dosieranschläge an dem Ende der Drehanschläge gebildet sein.

Die Fördereinrichtung ist vorzugsweise für eine manuelle Betätigung ausgebildet. Sie kann aber auch einen motorischen Antrieb umfassen, der die Förderbewegung bewirkt und in der Auslöseposition der Fördereinrichtung ausgelöst wird. In beiden Ausbildungen umfasst sie ein Betätigungselement, im einen Fall für die manuelle Betätigung und Bewirkung der Förderbewegung und im anderen Fall für das Auslösen des motorischen Antriebs. Für die manuelle Betätigung, wie sie für Injektionsgeräte bevorzugt wird, bringt der Verwender mittels des Betätigungselements die für die Förderbewegung erforderliche Förderkraft auf.

Vorteilhafterweise ist eine Weglänge, die das Betätigungselement bei der Betätigung ausführt, größer als eine Weglänge der Förderbewegung, die die Fördereinrichtung für eine vollständige Ausschüttung der eingestellten Produktdosis zurücklegt. Die Weglängenvergrößerung ist insbesondere dann von Vorteil, wenn die der eingestellten Produktdosis entsprechende Weglänge der Förderbewegung sehr kurz ist, beispielsweise einen oder wenige Millimeter oder gar weniger als einen Millimeter beträgt. Würde sich die Bewegung des Betätigungselements über eine ebenso kurze Weglänge nur erstrecken, wären Fehlinterpretationen dergestalt möglich, dass der Verwender glaubt, er habe die Produktdosis noch gar nicht oder nur unvollständig verabreicht.

Der gegenüber der Förderbewegung verlängerte Betätigungsweg des Betätigungselements kann getriebetechnisch so realisiert sein, dass die Bewegung des Betätigungselements mittels eines Untersetzungsgetriebes stets und kontinuierlich in die Förderbewegung untersetzt wird. Nicht zuletzt aus Gründen der Einfachheit umfasst der Betätigungsweg des Betätigungselements jedoch eine Leerbewegung des Betätigungselements ohne Förderbewegung und eine Bewegung des Betätigungselements gemeinsam, 1:1, mit der Förderbewegung.

In einer weiteren Ausführungsform umfasst das Antriebs- und Dosiermodul ein Dosierglied, das für die Einstellung der Produktdosis relativ zu einem Antriebsglied in eine von mehreren Dosierpositionen bewegbar ist, wobei eines aus Antriebsglied und Dosierglied mehrere Dosieranschläge auf axial unterschiedlichen Höhen und den Dosieranschlägen zugeordnete Drehanschläge, und das andere aus Antriebsglied und Dosierglied wenigstens ein Auswahlelement bilden. Das Antriebsglied ist translatorisch relativ zu dem Dosierglied bis in eine Auslöseposition bewegbar, in der das wenigstens eine Auswahlelement einem der Dosieranschläge in Anschlag ist. In der Auslöseposition sperrt das wenigstens eine Auswahlelement mit den Drehanschlägen die Drehung des Dosierglieds in beide Drehrichtungen.

Das Dosierglied entspricht einer Dosiereinrichtung. Das Dosierglied kann insbesondere hülsenförmig sein und ist für die Einstellung der Produktdosis relativ zu dem Antriebsglied drehbar. In Abhängigkeit von der Drehwinkelstellung können so mehrere Dosierpositionen eingestellt werden. Vorzugsweise umfasst das Dosierglied mehrere Dosieranschläge, die an der äußeren Umfangsfläche und bei einem hülsenförmigen Dosierglied an der inneren Umfangsfläche der Hülse verteilt angeordnet sein können. Die Dosieranschläge sitzen in Axialrichtung auf unterschiedlichen Positionen, so dass das Antriebsglied je nach Dosiereinstellung um Wege unterschiedlicher Längen in Axialrichtung bewegt werden kann. Vorzugsweise ist das Dosierglied für die Einstellung der Produktdosis relativ zu dem Abtriebsglied in eine von mehreren Dosierpositionen drehbar, wobei das Antriebsglied mit dem Abtriebsglied mechanisch gekoppelt und relativ zu dem Abtriebsglied translatorisch bewegbar ist. Vorteilhaft hängt die Produktdosis von den axial unterschiedlichen Höhen der mehreren Dosieranschläge ab. Die Dosieranschläge können über den Umfang verteilt angeordnet sein.

Die Dosieranschläge können jeweils durch eine Stirnseite einer Nut gebildet sein, wobei sich die Nut axial von einer Stirnseite des die Dosieranschläge enthaltenen Bauteils in das Bauteil erstreckt. Vorteilhaft ist die Nut in ihrer Längsrichtung halbseitig offen, so dass das Auswahlelement in die Nut eingerückt oder hineinbewegt werden kann. Die Nuten können an einer inneren Umfangsfläche einer Hülse oder auf einer äußeren Seite eines in eine Hülse eingreifenden Elements gebildet sein. Besonders bevorzugt verlaufen die Nuten in Axialrichtung und weisen eine taschenförmige Form auf, so dass das Auswahlelement nur von der Innenseite her in die Nut eingreifen kann. Die taschenförmigen Nuten weisen eine Tiefe auf, die geringer ist als die Wandstärke der Hülse. Grundsätzlich wäre es möglich, die Nuten durchgängig zu gestalten, so dass ihre Tiefe der Wandstärke der Hülse entspricht. Die Nuten können sich beginnend von der Stirnseite oder axial beabstandet von der Stirnseite des die Dosieranschläge enthaltenden Bauteils bis zu den Dosieranschlägen erstrecken.

Zwischen den vorteilhafterweise über den Umfang angeordneten Nuten verbleiben als Drehanschläge dienende Stege. Insbesondere werden die Drehanschläge durch die Flanken der Nut oder der Nuten gebildet.

Besonders bevorzugt sind an einem aus Antriebsglied und Dosierglied die auf das andere axial zuweisenden Dosieranschläge treppenförmig angeordnet. Auch hier können die Flanken der Nuten die Drehanschläge bilden. Zwischen jeder Stufe der Treppe sollte sich ein Drehanschlag befinden. Grundsätzlich ist es auch möglich zwischen manchen Stufen keinen Steg vorzusehen. Die Nut kann an Ihrer Stirnseite auch zwei oder mehrere Dosieranschläge bilden. Die über die Umfangsrichtung treppenförmig angeordneten Dosieranschläge können stetig steigend oder stetig fallend sein, sind aber nicht darauf beschränkt. In dieser Ausführungsform ist es besonders bevorzugt, dass die Dosieranschläge durch das Dosierglied und das wenigstens eine Auswahlelement durch das Abtriebsglied gebildet werden. Das wenigstens eine Auswahlelement kann ein Nocken oder eine in Axialrichtung verlaufender Steg sein, der vorzugsweise von einer Mantelfläche des Antriebsglieds oder des Dosierglieds abragt. Besonders vorteilhaft sind drei Auswahlelemente.

Insbesondere kann ein Mechanikhalter vorgesehen sein, mit dem das Dosierglied mechanisch gekoppelt ist, wobei das Dosierglied relativ zu dem Mechanikhalter drehbar ist. Ferner kann auch das Antriebsglied mechanisch mit dem Mechanikhalter gekoppelt sein, wobei das Antriebsglied relativ zu dem Mechanikhalter translatorisch bewegbar ist.

In einer weiteren Ausführungsform umfasst das Antriebs- und Dosiermodul ein Dosierglied, einen Mechanikhalter relativ zu dem das Dosierglied drehbar ist, wobei an einem aus Mechanikhalter und Dosierglied mindestens ein Sperrelement und dem anderen aus Mechanikhalter und Dosierglied mindestens ein Sperr-Gegenelement gebildet ist, in das das Sperrelement eingreifen und die Drehung des Dosierglieds relativ zu dem Mechanikhalter sperren kann. Vorzugsweise ist das Sperrelement in Radialrichtung federnd angeordnet. Die federnde Anordnung kann über ein Federelement erzeugt werden. Das Federelement kann beispielsweise eine Feder sein. Vorzugsweise wird die Feder durch einen Abschnitt des Bauteils gebildet, das das Sperrelement umfasst. Besonders bevorzugt sind das mindestens eine Sperrelement, das mindestens eine jeweils zu dem mindestens einen Sperrelement zugehörige Federelement und eines aus Mechanikhalter und Dosierglied einstückig. Besonders bevorzugt ist das mindestens eine Sperrelement an dem Dosierglied gebildet, wobei das mindestens eine Sperr-Gegenelement an dem Mechanikhalter gebildet ist.

Die Anzahl der Sperr-Gegenelemente kann größer oder gleich der Anzahl der Sperrelemente sein. Besonders bevorzugt entspricht die Anzahl der Sperr-Gegenelemente der Anzahl oder einem Vielfachen der Anzahl der Dosieranschläge. Die Anzahl der Sperr-Gegenelemente kann auch nur einen Bruchteil der Anzahl der Dosieranschläge entsprechen. Grundsätzlich sollten die Sperr-Gegenelemente über die Umfangsrichtung verteilt angeordnet sein. Es kann auch vorteilhaft sein, die Sperr-Gegenelemente abschnittsweise über den Umfang anzuordnen. Vorteilhaft weist eines aus Dosierglied und Antriebsglied mehrere über den Umfang angeordnete Dosieranschläge auf, wobei mehrere Sperr-Gegenelemente die gleiche Winkelteilung zueinander haben wie die Dosieranschläge zueinander. Das mindestens eine Sperrelement sollte mit den Dosieranschlägen und/oder den dazugehörigen Nuten in so einem Positionsverhältnis stehen, dass mindestens ein Sperrelement in je ein Sperr-Gegenelement einrücken kann, wenn das mindestens eine Auswahlelement in eine Nut des die Dosieranschläge enthaltenden Bauteils bewegt wird. Die Winkelteilung der Dosieranschläge und somit auch die der Sperr-Gegenelemente kann gleichmäßig sein. Eine ungleichmäßige Winkelteilung wäre jedoch auch denkbar.

Vorzugsweise kann ein Blockierelement das Sperrelement in einem Sperreingriff mit dem Sperr-Gegenelement halten. Dadurch wird die Drehung des Dosierglieds relativ zu dem Mechanikhalter gesperrt. In einer Ausführungsform kann das Sperrelement von den Sperr-Gegenelementen ausgerückt sein und durch das Blockierelement in das Sperr-Gegenelement eingerückt werden, so dass die Drehung des Dosierglieds relativ zu dem Mechanikhalter gesperrt wird. In einer anderen Ausführungsform ist das Sperrelement in das Sperr-Gegenelement eingerückt ohne, dass das Blockierelement das Sperrelement in dem Sperreingriff hält. Bei Drehung des Dosierglieds wird das Sperrelement aus dem Sperr-Gegenelement herausgedrückt und rastet bei weiterer Drehung in das nächste oder benachbarte Sperr-Gegenelement ein. Dies wird so lange vollzogen, bis durch die Drehung des Dosierglieds die gewünschte Dosis eingestellt ist. Durch das Blockieren des Sperrelements mit dem Blockierelement kann das Sperrelement nicht mehr aus dem Sperr-Gegenelement ausrücken, wodurch die Drehung des Dosierglieds relativ zu dem Mechanikhalter gesperrt wird. Vorzugsweise wird das Blockierelement translatorisch in einen Blockiereingriff mit dem Sperrelement gebracht. Denkbar wäre auch, dass der Blockiereingriff durch eine Drehbewegung oder einer Kombination aus einer Drehbewegung und einer Translationsbewegung erzeugt wird. Vorzugsweise wird das Blockierelement an einem oder von einem Betätigungselement gebildet. Besonders bevorzugt ist das Blockierelement als ein radial abstehender umlaufender Kragen oder als ein Absatz einer Hülse gebildet. Insbesondere soll das Blockierelement das Sperrelement zumindest in einer Freigabeposition freigeben. Das oder die Sperrelemente sind so lange blockiert, wie das Blockierelement ihnen gegenüberliegt. Das Blokkierelement kann sich über eine so große Länge in Längsrichtung erstrecken, dass dadurch die Drehung über einen langen Herausziehweg des Betätigungselements verhindert wird. Denkbar wäre auch, dass das Blockierelement nur so lange ist, dass es die Drehung bei der geringst möglichen Dosis oder bei den ersten geringst möglichen Dosen verhindert. Insbesondere könnte das Blockierelement das Sperrelement blockieren, wenn bei einem oder mehreren Auswahlelementen nicht alle Auswahlelemente in die Nuten eingerückt sind. Das Blockierelement könnte eine axiale Länge aufweisen, die kleiner ist als der Weg um den das Blockierelement bei einer maximal eingestellten Produktdosis translatorisch bewegt werden kann minus das Doppelte der Axiallänge des Sperrelements. Dabei würde das Blockierelement das Sperrelement bei einer hohen Dosis, spätestens jedoch bei der maximal möglichen Dosis freigeben. Das Blockierelement könnte das Sperrelement auch dann freigeben, wenn bei einem oder mehreren Auswahlelementen alle Auswahlelemente jeweils in eine Nut eingreifen. Die Blockierung der Drehung würde dann beispielsweise über alle in die Nut oder die Nuten bewegten Auswahlelemente erfolgen. Dadurch könnte das Antriebs- und Dosiermodul in seiner Baulänge verkürzt werden.

In einer weiteren Ausführungsform kann das wenigstens eine Auswahlelement mehrteilig sein. Denkbar wären zwei oder mehrere axial hintereinander angeordnete Auswahl-Teilelemente. Wenigstens eines der Auswahl-Teilelemente kann zur Dosisauswahl in Anschlag mit einem Dosieranschlag gebracht werden. Ein anderes der Auswahl-Teilelemente kann zur Verdrehsicherung dienen.

In einer anderen Ausführungsform ist mindestens eine die Drehanschläge bildende Nut von dem Dosieranschlag beabstandet. Prinzipiell könnte die Nut oder die die Flanken der Nut bildenden Stege in ihrer axialen Ausdehnung unterbrochen sein. Die Stege können wie eine axiale Führung wirken. Ein Auswahl-Teilelement bildet z. B. mit den Flanken der Nut eine Verdrehsicherung, wobei das andere Auswahl-Teilelement zur Dosisauswahl mit dem Dosieranschlag in einen axialen Anschlag gebracht wird.

Besonders bevorzugt kann das Antriebs- und Dosiermodul mit den oben beschriebenen verschiedenen Mechanismen ausgestattet sein, die die Dosierung einer Produktdosis außerhalb der Freigabeposition verhindern.

Die Merkmale der Erfindung können beliebig miteinander kombiniert werden.

Vorteilhafte Ausgestaltungen werden auch in den Unteransprüchen und deren Kombinationen beschrieben.

Bevorzugte Ausführungsbeispiele der Erfindung werden nachfolgend anhand von Figuren beschrieben. An den Ausführungsbeispielen offenbar werdende Merkmale bilden je einzeln und in jeder Merkmalskombination die Gegenstände der Ansprüche und auch die vorstehend beschriebenen Ausgestaltungen vorteilhaft weiter. Es zeigen:
- Figur 1: ein Injektionsgerät aus dem Stand der Technik und
- Figur 2: eine perspektivische Ansicht einer erfindungsgemäßen Dosiereinrichtung,
- Figur 3: eine perspektivische Ansicht eines erfindungsgemäßen Antriebsglieds,
- Figuren 4 bis 9: das erfindungsgemäße Zusammenwirken von Dosiereinrichtung und Antriebsglied,
- Figur 10: eine perspektivische Ansicht einer Dosiereinrichtung,
- Figur 11: ein erfindungsgemäßes Sperrelement, und
- Figuren 12 bis 15: ein erfindungsgemäßes Antriebs- und Dosiermodul.

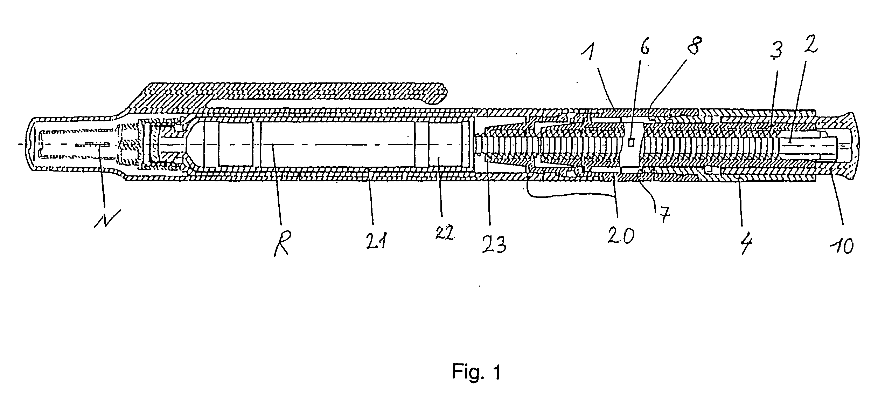

Figur 1 zeigt ein Injektionsgerät, das als Zahnstangenpen gebildet ist. Das Injektionsgerät umfasst ein zweiteiliges Gehäuse 20 aus einem distalen Gehäuseabschnitt und einem proximalen Gehäuseabschnitt, die miteinander fest verbunden sind, beispielsweise verschraubt. In einem Aufnahmefach des Gehäuses 20, das dessen distaler Gehäuseabschnitt bildet, ist ein Reservoir 21 aufgenommen. An einem distalen Auslass des Reservoirs 21 ist eine Injektionsnadel N befestigt. Die Längsachse der Injektionsnadel N bildet eine zentrale Längsachse R des Injektionsgeräts. Ein Kolben 22 verschließt das proximale Ende des Reservoirs 21. Der Kolben 22 kann entlang der Achse R auf den Auslass des Reservoirs 21 zu eine Förderbewegung ausführen, um Produkt aus dem Reservoir 21 zu verdrängen. Das Reservoir 21 ist eine handelsübliche Ampulle, die mit dem zu verabreichenden Produkt, beispielsweise Insulin, gefüllt ist.

Der Kolben 22 ist das unmittelbar auf das Produkt wirkende Förderglied einer Fördereinrichtung, die über den Kolben 22 hinaus ein Abtriebsglied 2, ein Antriebsglied 3 und ein Betätigungselement 10 umfasst. Wegen der Ausbildung des Förderglieds als Kolben 22 ist das unmittelbar auf den Kolben 22 wirkende Abtriebsglied 2 eine Kolbenstange und wird deshalb im Folgenden auch so bezeichnet. Die Kolbenstange 2 führt bei Betätigung der Fördereinrichtung 2; 3; 10; 22 ebenfalls die Förderbewegung aus und drückt dabei den Kolben 22 in die distale Richtung. Die Kolbenstange 2 ist als Zahnstange gebildet mit mehreren in Richtung der Achse R sich erstreckenden Zahnreihen, die zur Verfeinerung der Dosisauswahl entlang der Achse R je um weniger als eine Zahnteilung zueinander versetzt sind. Das Antriebsglied 3 ist entlang der Achse R in die distale und die proximale Richtung bewegbar. Das Antriebsglied 3 und die Kolbenstange 2 sind so miteinander gekoppelt, dass das Antriebsglied 3 bei seiner Bewegung in die distale Richtung die Kolbenstange 2 mitnimmt, die Bewegung in die proximale Richtung jedoch ohne die Kolbenstange 2 ausführt. Die Kopplung wird im Ausführungsbeispiel durch Eingriff von Mitnehmern in die Zahnreihen der Kolbenstange 2 bewirkt. Der Eingriff ist so, dass eine Bewegung der Kolbenstange 2 in die distale Richtung relativ zu dem Antriebsglied 3 verhindert und eine Bewegung des Antriebsglieds 3 in die proximale Richtung relativ zu der Kolbenstange 2 zugelassen wird. Um eine Mitnahme der Kolbenstange 2 bei der Bewegung in die proximale Richtung zu verhindern, bildet der proximale Abschnitt des Gehäuses 20 eine Rückhalteeinrichtung 23, die wie die Mitnehmer des Antriebsglieds 3 in wenigstens eine, im Ausführungsbeispiel in zwei Zahnreihen der Kolbenstange 2 eingreift, so dass die Kolbenstange 2 relativ zu dem Gehäuse 20 zwar in die distale Richtung, aber nicht in die proximale Richtung bewegt werden kann. Erreicht wird dies durch die Formung der Zähne der Zahnreihen als Sägezähne. Der proximale Abschnitt des Gehäuses 20 lagert die Kolbenstange 2 und das Antriebsglied 3 ferner so, dass diese Bestandteile der Fördereinrichtung 2; 3; 10; 22 relativ zu dem Gehäuse 20 keine Drehbewegungen um die Achse R ausführen können. Da der proximale Abschnitt des Gehäuses 20 wenigstens teilweise die Dosier- und Verabreichungsmechanik umfasst, kann er auch als Mechanikhalter 1 bezeichnet werden.

Das Injektionsgerät erlaubt pro Injektion eine freie Auswahl der verabreichbaren Produktdosis. Für die Auswahl bzw. Einstellung der Produktdosis ist ein Dosierglied 4 vorgesehen, das relativ zu der Fördereinrichtung 2; 3; 10; 22, insbesondere relativ zu deren Antriebsglied 3, eine Dosierbewegung ausführen kann. Der proximale Abschnitt des Gehäuses 20 lagert auch das Dosierglied in einer für die Ausführung der Dosierbewegung geeigneten Weise. Im Ausführungsbeispiel, in dem die Dosierbewegung eine Drehbewegung um die Achse R ist, lagert der Mechanikhalter 1 des Gehäuses 20 die Dosiereinrichtung 4 um die Achse R drehbar. Die Achse R bildet somit die Translationsachse für die Fördereinrichtung 2; 3; 10; 22 und die Rotationsachse für das Dosierglied 4. Das Dosierglied 4 ist bei Ausführung der Dosierbewegung zwischen diskreten, als Rastpositionen vorgegebenen Dosierpositionen bewegbar. Hierfür ist sie mit dem proximalen Abschnitt des Gehäuses 20 in jeder der Dosierpositionen in einem lösbaren Rasteingriff. Zu dem Dosierglied 4 sei noch angemerkt, dass es im Ausführungsbeispiel als Hülsenkörper gebildet ist und das Antriebsglied 3 sowie das Betätigungselement 10 umgibt. Das Antriebsglied 3 und das Betätigungselement 10 sind ebenfalls je als Hülsenkörper gebildet, wobei das Betätigungselement 10 einen proximalen Endabschnitt des Antriebsglieds 3 umgibt und für eine manuelle Betätigung der Fördereinrichtung 2; 3; 10; 22 in die proximale Richtung aus dem Dosierglied 4 hinausragt. Das Antriebsglied 3 schließlich umgibt die Kolbenstange 2.

Für die Einstellung der Produktdosis bilden das Antriebsglied 3 ein Auswahlelement 6 und das Dosierglied 4 einen Dosieranschlag 8, der dem Auswahlelement 6 in proximaler Richtung gegenüberliegt. Das Dosierglied 4 bildet ihren Dosieranschlag 8 mit einer distalen Stirnfläche, die jeweils zwei sich von der distalen Stirnfläche 7 des Dosierglieds 4 erstreckenden Drehanschläge 5 begrenzt. Das Antriebsglied 3 bildet sein Auswahlelement 6 als radial nach außen abragenden Nocken, dessen Form der Breite einen durch zwei Drehanschläge gebildeten Nut 9 bzw. der Breite des Dosieranschlags 8 angepasst ist.

In dem in Figur 1 dargestellten Zustand nimmt die Fördereinrichtung 2; 3; 10; 22 im Gehäuse 20 eine distalste Position ein. In diesem Zustand wird die Produktdosis mittels des Dosierglieds 4 eingestellt, indem ein der gewünschten Produktdosis entsprechender Dosieranschlag 8 in die entlang der Achse R dem Auswahlelement 6 gegenüberliegende Dosierposition bewegt wird. Der in der betreffenden Dosierposition zwischen Dosieranschlag 8 und Auswahlelement 6 und 27 verbleibende, entlang der Achse R gemessene Abstand entspricht der Weglänge, d. h. dem Förderhub, die das Antriebsglied 3 gemeinsam mit der Kolbenstange 2 und dem Kolben 22 bei der Injektion zurücklegen kann. Nach der Einstellung der Produktdosis wird durch Ziehen an dem Betätigungselement 10 das Antriebsglied 3 und wegen des Eingriffs damit gemeinsam die Kolbenstange 2 in die proximale Richtung gezogen, bis das Auswahlelement 6 in Kontakt mit dem Dosieranschlag 8 gelangt. Die Fördereinrichtung 2; 3; 10; 22 nimmt dann eine Auslöseposition ein, aus der heraus sie für die Injektion durch Ausübung einer in die distale Richtung auf das Betätigungselement 10 wirkenden Druckkraft betätigt werden kann, Es ist klar, dass vor der Injektion die in Figur 1 dargestellte Gehäusekappe und ferner die Nadelschutzkappe entfernt werden müssen.

Der proximale Abschnitt des Gehäuses 20, die von diesem Abschnitt gelagerten Teile der Fördereinrichtung 2; 3; 10; 22 und das mit dem Gehäuseabschnitt von der Dosierbewegung abgesehen fest verbundene Dosierglied 4 bilden ein Antriebs- und Dosiermodul, wie es aus der DE 199 00 792 C2 bekannt ist. Dieses Modul kann durch ein erfindungsgemäßes Antriebs- und Dosiermodul ersetzt werden.

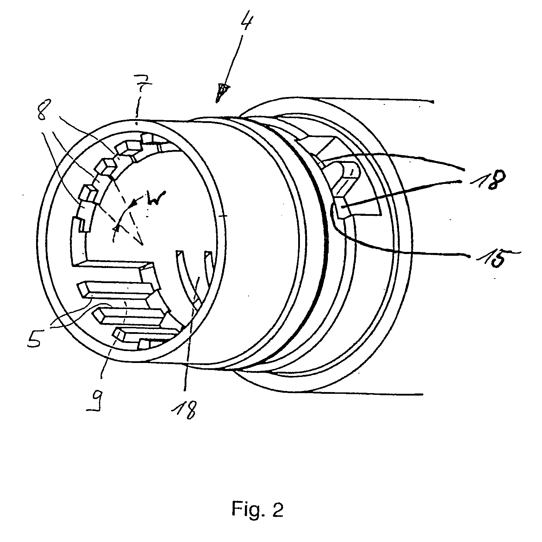

Figur 2 zeigt ein erfindungsgemäßes Antriebs- und Dosiermodul in einem ersten Ausführungsbeispiel. Teile gleicher Funktion wie die des Antriebs- und Dosiermoduls des bekannten Injektionsgeräts sind mit den gleichen Bezugszeichen versehen. Soweit Ausführungen zum erfindungsgemäßen Antriebs- und Dosiermodul nicht gemacht werden, kann das Modul insbesondere dem des Injektionsgeräts der Figur 1 entsprechen.

Das Antriebs- und Dosiermodul der Erfindung weist insbesondere eine Dosiersicherung in Form mehrerer Drehanschläge 5 auf, die eine Verstellung der eingestellten Produktdosis in der Auslöseposition der Fördereinrichtung verhindert. Die Dosiersicherung wird durch einen Eingriff zwischen der Fördereinrichtung und dem Dosierglied 4 gebildet, der in der Auslöseposition der Fördereinrichtung Dosierbewegungen des Dosierglieds 4 relativ zu der Fördereinrichtung blockiert und deshalb im Folgenden als Blokkiereingriff bezeichnet wird.

Figur 2 zeigt eine erfindungsgemäße Dosiereinrichtung 4 in Form eines Dosierglieds 4. Das Dosierglied 4 ist hülsenförmig aufgebaut. Die Außenseite der Hülse weist mehrere Absätze auf, die unter anderem als Greiffläche oder als Element dienen, mit dem eine Kopplung mit einem Mechanikhalter 1 möglich ist. An der Innenseite der Hülse sind Dosieranschläge 8 angebracht. Die Dosieranschläge 8 sind auf unterschiedlichen axialen Höhen angebracht. Insbesondere sind die Dosieranschläge 8 treppenförmig über den Umfang angeordnet. In dem gezeigten Beispiel ist die Treppe stetig fallen bzw. stetig steigend. Der Winkel zwischen einem Dosieranschlag 8 und dem nächsten Dosieranschlag 8 oder einem anderen beliebigen Dosieranschlag 8 wird als Winkelteilung W bezeichnet. Die Winkelteilung W und/oder die Anzahl der Dosieranschläge 8 sollten z. B. denen der Sperr-Gegenelemente 16 in dem Mechanikhalter 1 entsprechen.

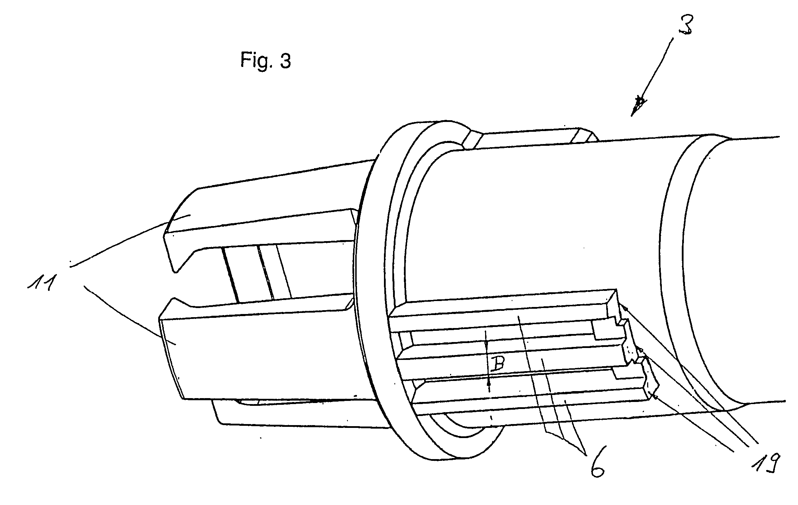

Zwischen den Treppenstufen bzw. den Dosieranschlägen sind in Längsrichtung verlaufende Stege angeordnet, die mit ihren Flanken Drehanschläge bilden. Die Kombination aus zwei Stegen und mindestens einem Dosieranschlag 8 kann auch als Nut 9 bezeichnet werden, wobei die Nut 9 halbseitig offen ist und die Flanken der Nut 9 die Drehanschläge 5 bilden. Die die Drehanschläge 5 enthaltenen Stege ragen von jeweils einem Dosieranschlag 8 in Längsrichtung R ab, wobei die Höhe und die Breite des Stegs geringer sind als die Höhe und die Breite des dazugehörigen Dosieranschlags. Durch diese Gestaltung eines Stegs mit dem Dosieranschlag kann eine Stirnfläche 19 (Figur 3) eines Auswahlelementes 6 stabiler und verschleißfester gestaltet werden. Der Abstand zwischen zwei eine Nut 9 bildenden Flanken 5 ist größer als die Breite B eines Auswahlelements 6.

Die an der offenen Stirnseite einer Nut 9 gebildeten Stirnflächen eines Stegs sind von einer Stirnseite 7 des Dosierglieds 4 axial in das Dosierglied 4 versetzt.

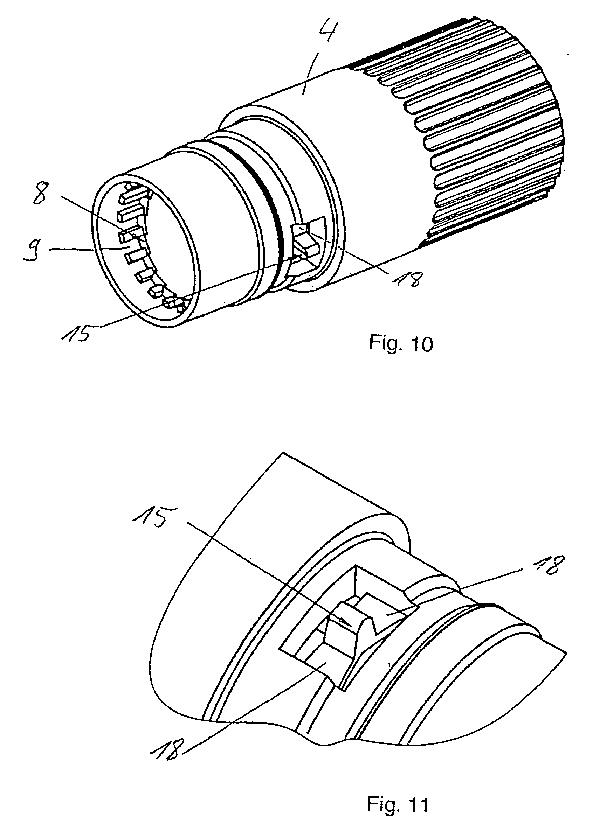

Die Figuren 2, 10 und 11 zeigen insbesondere zwei Sperrelemente 15, die diametral gegenüberliegend, in Radialrichtung federnd an dem Dosierglied 4 angeordnet sind. Das Sperrelement 15 ist in einem fensterartigen Durchbruch des Dosierglieds 4 angeordnet. Das Dosierglied 4 bildet zwei Federelemente 18, die jeweils mit dem Sperrelement 15 verbunden sind. Durch die konstruktive Ausgestaltung der Federelemente 18 kann das Sperrelement 15 in Radialrichtung federn. In dem gezeigten Beispiel sind Sperrelemente 15, Federelemente 18 und Dosierglied 4 einstückig. Das Sperrelement 15 ist an seiner radial nach außen weisenden Seite abgerundet, um besser von einem zum nächsten oder benachbarten Sperr-Gegenelement 16 aus- und einrasten zu können. Durch die Dosiseinstellbewegung des Dosierglieds 4 wird das Sperrelement 15 durch deren vorteilhafte Ausgestaltung aus dem jeweiligen Sperr-Gegenelement 16 herausgedrückt und rastet in Folge der Federwirkung der Federelemente 18 in das nächste Sperr-Gegenelement 16 ein.

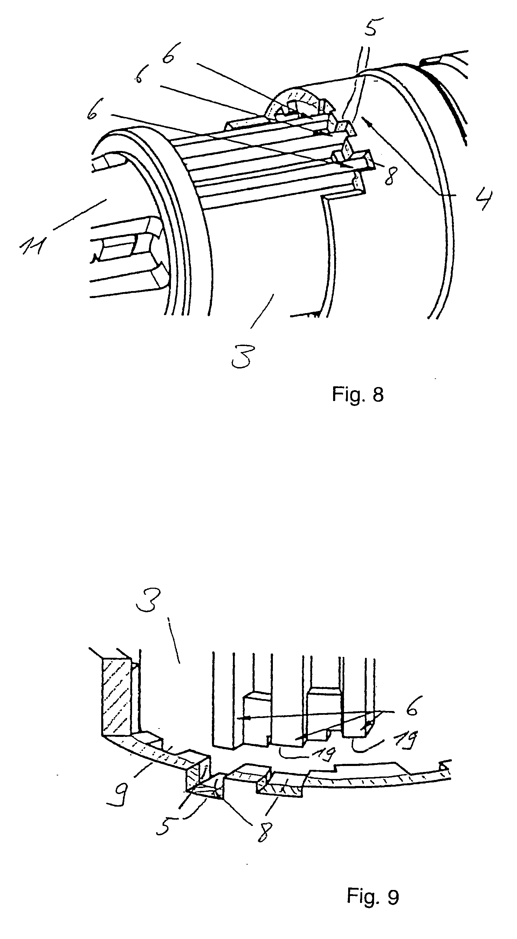

Figuren 3 und 9 zeigen ein erfindungsgemäßes Antriebsglied 3. Das Antriebsglied 3 weist an seinem distalen Ende mehrere Rückhalteeinrichtungen 11 für ein Abtriebsglied 2 auf. Insbesondere ist an der äußeren Umfangsfläche des Antriebsglieds 3 mindestens ein Auswahlelement 6 angebracht. Wie aus Figur 3 ersichtlich ist, ist es besonders vorteilhaft drei Auswahlelemente 6 an dem Antriebsglied anzuordnen. Die Form der Stirnflächen 19 der Auswahlelemente 6 entspricht in etwa dem Spiegelbild der Dosieranschläge 8. Die Auswahlelemente 6 sind in diesem Beispiel in Längsrichtung R verlaufende Stege, deren Breiten B angepasst sind, um in die Nuten 9 des Dosierglieds 4 bewegt werden zu können.

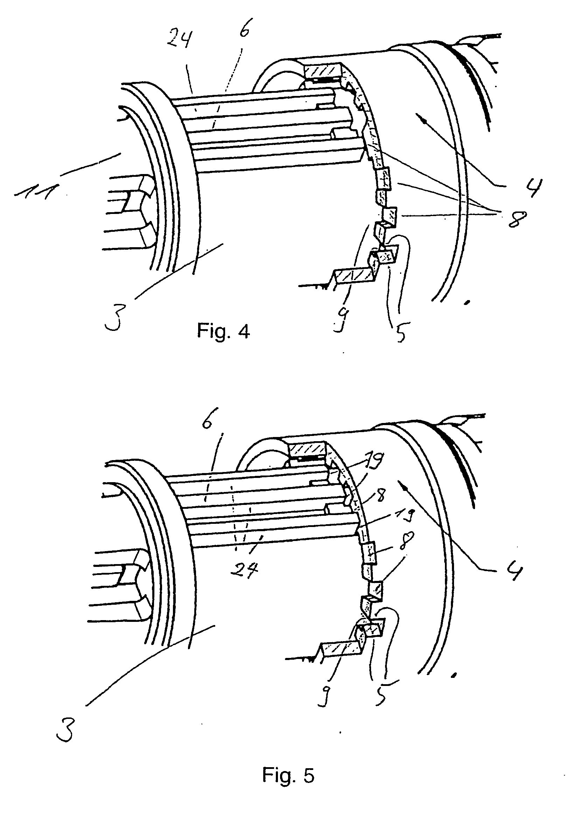

Figur 4 zeigt das Antriebsglied 3 und das Dosierglied 4 in einer Freigabeposition. Das Dosierglied 4 ist relativ zu dem Antriebsglied 3 drehbar. Keines der drei Auswahlelemente 6 greift in eine Nut 9 des Dosierglieds 4 ein.

Figur 5 zeigt das Dosierglied 4 und das Antriebsglied 3 in einer Auslöseposition für eine geringst mögliche Produktdosis. Die Stirnflächen 19 der Auswahlelemente 6 sind entsprechend den Dosieranschlägen 8 auch auf unterschiedlichen axialen Höhen angeordnet. Deshalb schlägt lediglich eine Stirnseite 19 an einem Dosisanschlag 8 an. Eine Verdrehsicherung findet bei diesem Beispiel nicht statt, da das Auswahlelement 6 in keine Nut 9 eingreift. Um eine Verdrehung des Dosierglieds 4 gegen das Antriebsglied 3 dennoch zu verhindern ist es besonders vorteilhaft, das Dosierglied 4 mit einem Sperrelement 15 zusätzlich verdrehzusichern.

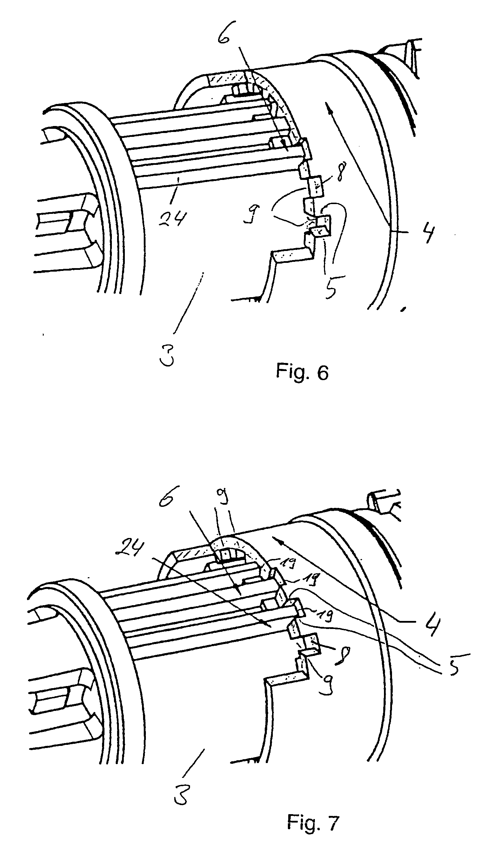

Figur 6 zeigt das Dosierglied 4 und das Antriebsglied 3 in einer Auslöseposition, bei der je eine Stirnfläche 19 von zwei Auswahlelementen 6 an je einem Dosieranschlag 8 anschlägt. Zusätzlich ist ein Auswahlelement 6 mit seinen Flanken 24 in dem Eingriff einer Nut 9, wodurch das Dosierglied 4 gegen das Antriebsglied 3 in beide Richtungen verdrehgesichert ist. Die Überlappung der Flanken 24 des Auswahlelements 6 mit den Drehanschlägen 5 ist jedoch nur sehr gering, so dass durch ein leicht erhöhtes Drehmoment an dem Dosierglied 4 eine Verdrehung des Dosierglieds 4 gegen das Antriebsglied 3 möglich sein könnte. Deshalb wird das Dosierglied 4 mit mindestens einem, vorzugsweise zwei Sperrgliedern 15 zusätzlich verdrehgesichert.

Figur 7 zeigt die Stirnflächen 19 der Auswahlelemente 6 in Anschlag mit jeweils einem Dosieranschlag 8. Zwei der drei Auswahlelemente 6 sind in die Nuten 9 eingerückt und bilden eine Verdrehsicherung. Die Überlappung der Flanken 24 mit den Drehanschlägen 5 ist im Vergleich zu Figur 6 größer. Auch hier wäre eine zusätzliche Verdrehsicherung mit Sperrelementen 15 sinnvoll.

In Figur 8 befindet sich das Antriebsglied 3 in einer Auslöseposition, wobei alle drei Auswahlelemente 6 in jeweils eine Nut 9 eingerückt sind und dadurch eine Verdrehsicherung in beide Richtungen bilden. Grundsätzlich steigt mit der eingestellten Produktdosis auch die Verdrehsicherheit des Dosierglieds 4 gegen das Antriebsglied 3. Folglich kann bei einer höheren Produktdosis die zusätzliche Verdrehsicherung der Sperrglieder 15 entfallen. Vorzugsweise sichern die Sperrelemente 15 die Verdrehung des Dosierglieds 4 gegen das Antriebsglied 3 nur so lange, bis alle Eingriffsglieder erstmalig jeweils in eine Nut eingreifen.

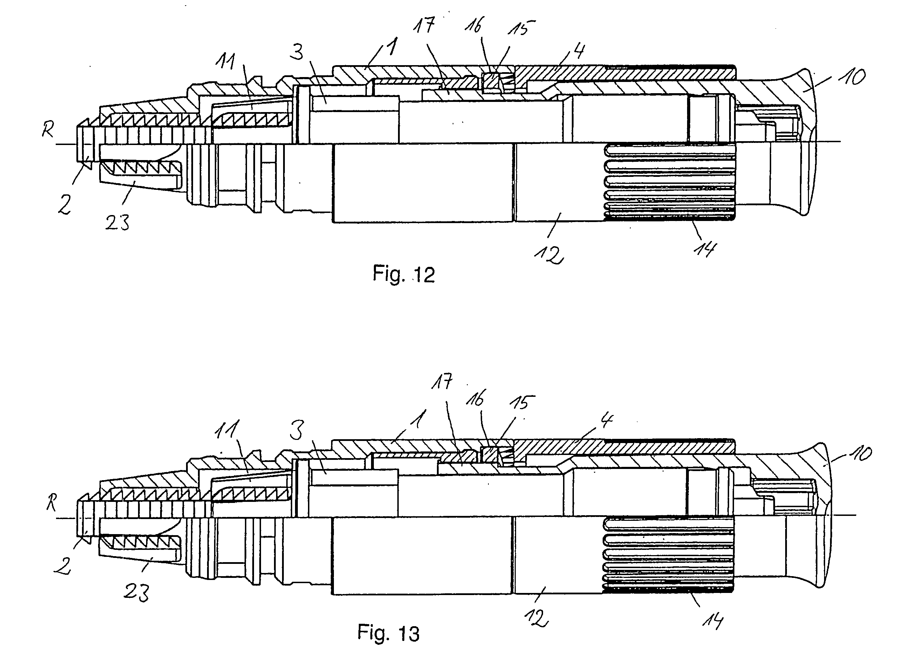

Figuren 12 bis 15 zeigen das Antriebs- und Dosiermodul zur Erfindung. Es umfasst einen Mechanikhalter 1, der auch als proximaler Gehäuseabschnitt 1 bezeichnet wird. Der Mechanikhalter 1 bildet an seinem vorderen Ende Rückhalteeinrichtungen 23, die schwingenartig an dem distalen Ende des Mechanikhalters 1 befestigt sind, so dass sie elastisch um ihren Befestigungspunkt hin- und hergebogen können. Die Rückhalteeinrichtung 23 greift in ein Abtriebsglied 2 ein. Das Abtriebsglied 2 ist vorzugsweise eine Vortriebsstange bzw. eine Stange, die eine sägezahnartige Verzahnung aufweist. Rückhalteeinrichtung 23 und Kolbenstange 2 greifen mit ihren sägezahnartig ausgebildeten Profilen ineinander. Die schrägen Flächen der Sägeverzahnung weisen bevorzugt in die distale Richtung, also die Richtung in die später der Kolben des Reservoirs bewegt wird.

Der Mechanikhalter 1 umschließt teilweise ein Dosierglied 4, und lagert es gleichzeitig. Das Dosierglied 4 ist insbesondere in dem Mechanikhalter 1 so gelagert, dass es nur rotatorische Bewegungen ausführen kann. Dies wird dadurch sichergestellt, dass sich an dem Mechanikhalter 1 Rastnasen befinden, die in eine radial umlaufende Nut des Dosierglieds 4 eingreifen und dadurch die Axialbewegung des Dosierglieds 4 relativ zu dem Mechanikhalter 1 verhindert. Das Dosierglied 4 kann aus mehreren absätzartigen Umfangsflächen bestehen. An dem proximalen Ende des Dosierglieds 4 befindet sich ein Abschnitt der eine Fläche bildet, die es einem Benutzer ermöglicht, das Dosierglied 4 gegenüber dem Mechanikhalter 1 zu drehen. Diese Fläche wird insbesondere als Greiffläche 14 bezeichnet. An einem weiteren Abschnitt des Dosierglieds 4 ist eine Skalentrommel 12 gebildet, an deren Umfangsseite sich eine Skala befindet, an der ein Benutzer der Vorrichtung die von ihm eingestellte Produktdosis ablesen kann. Dazu weist der Mechanikhalter 1 ein Fenster auf, das das Ablesen eines der Skalenwerte der Skala 13 ermöglicht.

Das Dosierglied 4 kann den bereits beschriebenen Dosiergliedern 4 entsprechen. Die Dosieranschläge 8 weisen in Bezug auf die Stirnseite 7 unterschiedliche axiale Abstände auf. Diese unterschiedlichen Abstände der Dosieranschläge 8 zu der Stirnseite 7 sind jeweils einer Produktdosis zugeordnet. Die Anzahl der Nuten 9 kann die Anzahl der einzelnen Elemente der Skala 13 bestimmen. Vorzugsweise sind Anzahl und Teilung der Elemente der Skala 13 gleich der der treppenförmigen Anordnung der Dosieranschläge 8.

Das Dosierglied 4 lagert das Antriebselement 3. Zwischen Antriebselement 3 und Dosierglied 4 ist bei der Dosierung eine rotatorische Relativbewegung möglich. Das Antriebsglied 3 wird ferner durch den Mechanikhalter 1 gelagert. Das Antriebsglied 3 kann relativ zu dem Mechanikhalter 1 nur translatorische Bewegungen ausführen. Dies wird sichergestellt durch ineinandergreifende Elemente, die durch den Mechanikhalter 1 und dem Antriebsglied 3 gebildet sind. Bevorzugt ist, dass der Mechanikhalter 1 einen Nocken oder eine andere abstehende Struktur aufweist, die in eine in dem Mechanikhalter 1 gebildete Nut eingreift. Die Nut ist in Axialrichtung orientiert. Dadurch wird die Drehbewegung des Antriebsglieds 3 relativ zu dem Mechanikhalter 1 verhindert, die Axialbewegung jedoch ermöglicht.

An dem distalen Ende des Antriebsglieds 3 befinden sich Rückhalteeinrichtungen 11, die ebenso wie die Zahnstange 2 eine sägezahnartige Verzahnung aufweisen, und in die Kolbenstange 2 eingreifen. Die Rückhalteelemente 11 sind schwingenartig ausgebildet und an der distalen Stirnseite des Antriebsglieds 3 befestigt. Die Rückhalteeinrichtungen 11 können um ihren Befestigungspunkt an dem Antriebsglied 3 elastisch hin- und herbewegt werden. Die Rückhalteeinrichtungen 11 des Antriebsglieds 3 entsprechen im wesentlichen denen der Rückhalteeinrichtungen 23 des Mechanikhalters 1.

Das Antriebsglied 3 weist an seinem proximalen Ende ein Verbindungselement auf, das die Verbindung mit einem Betätigungselement 10 erlaubt. Betätigungselement 10 und Antriebsglied 3 sind so miteinander verbunden, dass bei einem Herausziehen des Betätigungselements 10 aus dem Dosierglied 4 das Antriebsglied 3 mitgenommen wird.

Das Antriebsglied 3 kann dem beschriebenen Antriebsgliedern 3 entsprechen. Das Auswahlelement 6 weist solche Abmessungen auf, die es dem Auswahlelement 6 erlauben, in die Nuten 9 bewegt zu werden.

Wie bereits beschrieben sind die Sperrelemente 15 in Radialrichtung federnd gelagert und vorzugsweise ein integraler Bestandteil des Dosierglieds 4. An dem Mechanikhalter 1 sind mehrere, vorzugsweise über die Umfangsrichtung gleichmäßig verteilte Sperr-Gegenelemente 16 angeordnet. Da grundsätzlich zur Verdrehsicherung auch ein einziges Sperrelement 15 ausreichen würde, wird im Folgenden die Wirkungsweise des einen Sperrelements beschrieben, wobei dies jedoch auch für mehrere Sperrelemente 15 gilt. In der Freigabeposition ist das Dosierglied 4 gegen den Mechanikhalter 1 drehbar. In den in den Figuren 12 bis 15 beschriebenen Ausführungsformen befindet sich das Sperrelement 15 in der Freigabeposition in einem Rasteingriff mit einem Sperrgegenelement 16. Die Sperr-Gegenelemente sind beispielsweise Rastnuten, die dem Sperrelement 15 gegenüberliegen. Durch Drehung des Dosierglieds 4 wird das Sperrelement 15 in Folge der Drehbewegung aus dem jeweiligen Sperr-Gegenelement 16 radial nach innen herausgedrückt. Das Sperr-Gegenelement 16 rastet in das benachbarte Sperr-Gegenelement 16 ein. Ein Blockierelement 17 ist derart gestaltet, dass es die radial nach innen gerichtete Bewegung des Sperrelements 15 blockieren kann. Das Blokkierelement 17 ist ein an dem Betätigungselement 10 radial nach außen weisender Kragen. Zum Blockieren der Rastbewegung des Sperrelements 15 wird das Betätigungselement 10 in proximale Richtung bewegt, so dass das Blockierelement 17 in einem Bereich innerhalb des Sperrelements 15 bewegt wird und dadurch die Rastbewegung des Sperrelements 15 sperrt. Anders als dargestellt könnte das Sperrelement 15 in der Freigabeposition aus dem Sperr-Gegenelement 16 ausgerückt sein. Durch das Blokkierelement 17 könnte das Sperrelement 15 in eines der Sperr-Gegenelemente 16 eingerückt werden. Durch das Bewegen des Betätigungselements 10 in proximale Richtung würde das Blockierelement 17 das Sperrelement 15 radial nach außen drücken, so dass das Sperrelement 15 mit dem Sperr-Gegenelement 16 eine Verdrehsicherung in beide Drehrichtungen sicherstellen würde.

Zur Dosiseinstellung werden die Nuten 9 durch Drehung der Dosierhülse 4 relativ zu dem Antriebsglied 3 verdreht, bis die gewünschten Nuten 9 den Auswahlelementen 6 gegenüberliegen. Die Dosis bestimmt sich aus dem axialen Abstand zwischen den Dosieranschlägen 8 und dem Auswahlelementen 6. Durch die unterschiedlichen Tiefen der Nuten 9 bzw. durch die axiale Höhe der Dosieranschläge 8 wird die Produktdosis eingestellt. Wenn die gewünschten Nuten 9 den Auswahlelementen 6 gegenüberliegen, wird das Betätigungselement 10 relativ zu der Dosierhülse 4 herausgezogen, so dass die Auswahlelemente 6 in die gewünschten Nuten 9 bis zu den Dosieranschlägen 8 einfahren. Durch die Drehanschläge 5 wird eine Verstellung der Dosis mittels der Dosierhülse 4 verhindert. Durch das Herausziehen des Betätigungselementes 10 und des damit gekoppelten Antriebsglieds 3 führen die Rückhalteeinrichtungen 11 eine schwingende Bewegung aus, da ihre schräg zu der Längsachse verlaufenden Flächen aufeinander gleiten. In der Freigabeposition rasten die Rückhalteeinrichtungen in die Kolbenstange 2 ein. Die Kolbenstange 2 wird durch das Zurückbewegen des Betätigungselement 10 nicht zurückgezogen, da die Rückhalteeinrichtungen 23 ein Zurückziehen der Kolbenstange 2 verhindern.

Zur Verabreichung des Produkts wird das Betätigungselement 10 in distale Richtung gedrückt, so dass das damit gekoppelte Antriebsglied 3 die Rückhalteeinrichtungen 11 ebenfalls in distale Richtung bewegt. Durch die sägezahnartige Verzahnung der Rückhalteeinrichtung 11 wird das Abtriebsglied 2 in distale Richtung mitgenommen. Die Rückhalteeinrichtungen 23 des Mechanikhalters 1 rasten hierbei aufgrund der schrägen Flächen der Sägeverzahnung aus und erst dann wieder ein, wenn die Bewegung der Kolbenstange 2 beendet ist.

Figur 12 zeigt das Antriebs- und Dosiermodul in einer Freigabeposition vor einer Verabreichung. Das Blockierelement 17 befindet sich nicht in dem Bereich des Sperrelements 15, so dass das Sperrelement 15 radial nach innen gerichtete Rastbewegungen ausführen kann. Die Dosierung durch Drehung des Dosierglieds 4 ist möglich.

Figur 13 zeigt das Antriebs- und Dosiermodul bei dem Zurückziehen des Betätigungselements 10 in proximale Richtung. Das Blockierelement 17 ist in den Bereich des Sperrelements 15 bewegt und blockiert somit die radial nach innen gerichtete Rastbewegungen. Eine Dosierung ist nun nicht mehr möglich, da das Dosierglied 4 in beide Drehrichtungen gesperrt ist.

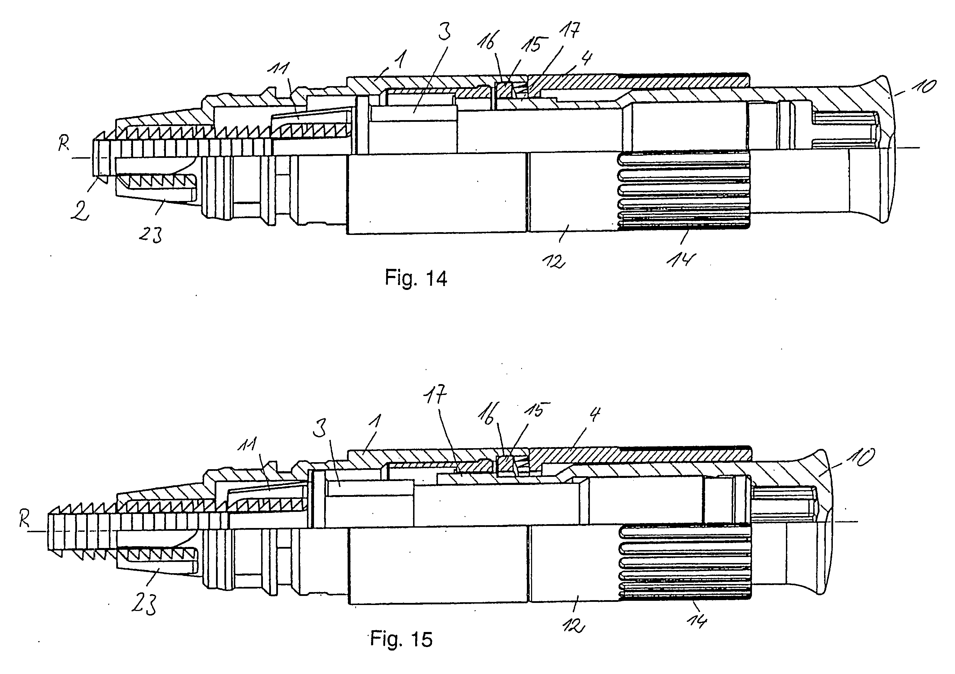

Figur 14 zeigt das Antriebs- und Dosiermodul in einer Auslöseposition, bei der das Sperrelement 15 blockiert ist. Ferner wurde das durch das Zurückziehen des Betätigungselements 10, das Antriebsglied 3 durch das Betätigungselement 10 mitgenommen. Das Antriebs- und Dosiermodul ist nun bereit für eine Verabreichung der eingestellten Produktdosis.

Figur 15 zeigt das Antriebs- und Dosiermodul in einer Freigabeposition nach der Verabreichung. Das Abtriebsglied 2 ist entsprechend der eingestellten Dosierung in distale Richtung bewegt worden. Das Sperrelement 15 ist von dem Blockierelement 17 wieder freigegeben, so dass eine erneute Dosierung mit dem Dosierglied 4 und eine anschließende Verabreichung einer weiteren Produktdosis möglich ist.

### Bezugszeichen

- 1: Mechanikhalter
- 2: Abtriebsglied, Vortriebsstange
- 3: Antriebsglied, Vortriebshülse
- 4: Dosierglied
- 5: Drehanschlag
- 6: Auswahlelement, Vorsprung
- 7: Stirnseite (des Bauteils)
- 8: Dosieranschlag
- 9: Nut
- 10: Betätigungselement
- 11: Rückhalteeinrichtung
- 12: Skalentrommel
- 13: Skala
- 14: Greiffläche
- 15: Sperrelement, Schnapper
- 16: Sperr-Gegenelement
- 17: Blockierelement
- 18: Federelement
- 19: Stirnfläche
- 20: Gehäuse
- 21: Reservoir
- 22: Kolben
- 23: Rückhalteeinrichtung
- 24: Flanke

- R: Rotationsachse, Längsachse
- N: Injektionsnadel
- B: Stegbreite
- W: Winkelteilung

## Patentansprüche

1. Antriebs- und Dosiermodul für eine Injektionsvorrichtung, umfassend:
a) ein Dosierglied (4), das für die Einstellung der Produktdosis relativ zu einem Antriebsglied (3) in eine von mehreren Dosierpositionen bewegbar ist,
b) wobei eines aus Antriebsglied (3) und Dosierglied (4) mehrere Dosieranschläge (8) auf axial unterschiedlichen Höhen und den Dosieranschlägen (8) zugeordnete Drehanschläge (5),
c) und das andere aus Antriebsglied (3) und Dosierglied (4) wenigstens ein Auswahlelement (6) bilden,
d) und wobei das Antriebsglied (3) translatorisch relativ zu dem Dosierglied (4) bis in eine Auslöseposition bewegbar ist, in der das wenigstens eine Auswahlelement (6) mit einem der Dosieranschläge (8) in Anschlag ist und
e) in der Auslöseposition das wenigstens eine Auswahlelement (6) mit den Drehanschlägen (5) die Drehung des Dosierglieds (4) in beide Drehrichtungen sperrt.

2. Antriebs- und Dosiermodul nach dem vorhergehenden Anspruch, **dadurch gekennzeichnet, dass** das Dosierglied (4) für die Einstellung der Produktdosis relativ zu einem Abtriebsglied (3) in eine von mehreren Dosierpositionen drehbar ist, wobei das Antriebsglied mit dem Abtriebsglied mechanisch gekoppelt und relativ zu dem Abtriebsglied translatorisch bewegbar ist.

3. Antriebs- und Dosiermodul nach einem der beiden vorhergehenden Ansprüche, **dadurch gekennzeichnet, dass** die Produktdosis von den axial unterschiedlichen Höhen der mehreren Dosieranschläge (8) abhängt.

4. Antriebs- und Dosiermodul nach einem der vorhergehenden Ansprüche, **dadurch gekennzeichnet, dass** die Dosieranschläge (8) durch jeweils eine Stirnseite (8) einer Nut (9) gebildet werden, die sich axial von einer Stirnseite (7) des die Dosieranschläge (8) enthaltenden Bauteils (3; 4) erstreckt.

5. Antriebs- und Dosiermodul nach dem vorhergehenden Anspruch, **dadurch gekennzeichnet, dass** die Drehanschläge (5) durch die Flanken (5) der Nut (9) gebildet werden.

6. Antriebs- und Dosiermodul nach einem der vorhergehenden Ansprüche, **dadurch gekennzeichnet, dass** an einem aus Antriebsglied (3) und Dosierglied (4) die auf das andere axial zu weisenden Dosieranschläge (8) treppenförmig angeordnet sind, wobei die Flanken (5) der Nuten die Drehanschläge (5) bilden.

7. Antriebs- und Dosiermodul nach einem der vorhergehenden Ansprüche, **dadurch gekennzeichnet, dass** die Dosieranschläge (8) durch das Dosierglied (4) und das wenigstens eine Auswahlelement (6) durch das Abtriebsglied (3) gebildet werden.

8. Antriebs- und Dosiermodul nach einem der vorhergehenden Ansprüche, **dadurch gekennzeichnet, dass** das wenigstens eine Auswahlelement (6) ein Nocken oder ein in Axialrichtung verlaufener Steg ist, die vorzugsweise von einer Mantelfläche des Antriebsglieds (3) oder des Dosierglieds (4) abragen.

9. Antriebs- und Dosiermodul nach einem der vorhergehenden Ansprüche, **dadurch gekennzeichnet, dass** das Dosierglied (4) mit einem Mechanikhalter (1) mechanisch gekoppelt ist, wobei das Dosierglied (4) relativ zu dem Mechanikhalter (1) rotierbar ist.

10. Antriebs- und Dosiermodul nach einem der vorhergehenden Ansprüche, **dadurch gekennzeichnet, dass** das Antriebsglied (3) mit einem Mechanikhalter (1) mechanisch gekoppelt ist, wobei das Antriebsglied (3) relativ zu dem Mechanikhalter (1) translatorisch bewegbar ist.

11. Antriebs- und Dosiermodul nach einem der vorhergehenden Ansprüche, **dadurch gekennzeichnet, dass** eine Fördereinrichtung (3-7) ein manuell betätigbares Betätigungselement (6) umfasst, dessen Betätigung die Förderbewegung bewirkt, und dass eine Weglänge (H_{F}+H_{L}) einer von dem Betätigungselement (6) bei Betätigung ausführbaren Bewegung größer ist als die der vollständigen Ausschüttung der eingestellten Produktdosis entsprechende Weglänge (H_{F}) der Förderbewegung.

12. Antriebs- und Dosiermodul nach dem vorhergehenden Anspruch, **dadurch gekennzeichnet, dass** die Fördereinrichtung (3-7) wenigstens ein in einem Reservoir (2) angeordnetes Förderglied (3) aufweist, das die Förderbewegung ausführt, und dass das Betätigungselement (6) mit dem Förderglied (3) so gekoppelt ist, dass es über einen Teil (H_{F}) der Weglänge (H_{F}+H_{L}) seiner Bewegung das Förderglied (3) mitnimmt und den anderen Teil (H_{L}) der Weglänge seiner Bewegung (H_{F}+H_{L}) ohne das Förderglied (3) zurücklegt.

13. Antriebs- und Dosiermodul für eine Injektionsvorrichtung, umfassend
a) ein Dosierglied (4),
b) einen Mechanikhalter (1) relativ zu dem das Dosierglied (4) drehbar ist,
c) wobei an einem aus Mechanikhalter. (1) und Dosierglied (4) mindestens ein Sperrelement (15) und
d) dem anderen aus Mechanikhalter (1) und Dosierglied (4) mindestens ein Sperr-Gegenelement (16) gebildet ist, in das das Sperrelement (15) eingreifen und die Drehung des Dosierglieds (4) relativ zu dem Mechanikhalter (1) sperren kann.

14. Antriebs- und Dosiermodul nach dem vorhergehenden Anspruch, **dadurch gekennzeichnet, dass** das Sperrelement (15) in Radialrichtung federnd angeordnet ist.

15. Antriebs- und Dosiermodul nach einem der zwei vorhergehenden Ansprüche, **dadurch gekennzeichnet, dass** das Sperrelement (15) an dem Dosierglied (4) gebildet ist.

16. Antriebs- und Dosiermodul nach einem der drei vorhergehenden Ansprüche, **dadurch gekennzeichnet, dass** die Anzahl der Sperr-Gegenelemente (16) größer oder gleich der Anzahl der Sperrelemente (15) ist.

17. Antriebs- und Dosiermodul nach einem der vier vorhergehenden Ansprüche, **dadurch gekennzeichnet, dass** eines aus Dosierglied (4) und Antriebsglied (3) mehrere über den Umfang angeordnete Dosieranschläge (8) aufweist, wobei mehrere Sperr-Gegenelemente (16) die gleiche Winkelteilung (W) zueinander haben wie die Dosieranschläge (8).

18. Antriebs- und Dosiermodul nach einem der fünf vorhergehenden Ansprüche, **dadurch gekennzeichnet, dass** ein Blockierelement (17) das Sperrelement (15) in einem Sperreingriff mit dem Sperr-Gegenelement (16) halten kann, wodurch die Drehung des Dosierglieds (4) relativ zu dem Mechanikhalter (1) gesperrt wird.

19. Antriebs- und Dosiermodul nach dem vorhergehenden Anspruch, **dadurch gekennzeichnet, dass** das Blockierelement (17) an einem oder von einem Betätigungselement (10) gebildet ist.

20. Antriebs- und Dosiermodul nach einem der Ansprüche 13 oder 19, **dadurch gekennzeichnet, dass** zumindest in einer Freigabeposition ein Blockierelement (17) das Sperrelement (15) freigibt.

21. Antriebs- und Dosiermodul nach einem der Ansprüche 13 bis 20, **dadurch gekennzeichnet, dass** ein Blockierelement (17) eine axiale Länge aufweist, die kleiner ist als der Weg, um den das Blockierelement (17) bei einer maximal eingestellten Produktdosis translatorisch bewegt werden kann minus das doppelte der axialen Länge des Sperrelements (15).

22. Antriebs- und Dosiermodul nach einem der Ansprüche 13 bis 21, **dadurch gekennzeichnet, dass** das Antriebs- und Dosiermodul mindestens eines der Merkmale der Ansprüche 1 bis 12 aufweist.

23. Injektionsgerät zum Injizieren einer einstellbaren Produktdosis umfassend ein Antriebs- und Dosiermodul, das mindestens einem der Ansprüche 1 bis 12 in Kombination mit mindestens einem der Ansprüche 13 bis 21 entspricht.

## Claims

1. Drive and metering module for an injection device, comprising:
a) a metering element (4), which can be moved relative to a drive element (3) to one of a number of metering positions for the adjustment of the product dose,
b) in which one of the many metering stops (8) of the drive element (3) and metering element (4) assembly, forms the rotation stops (5) assigned to the metering stops (8) at axially different heights,
c) and the other of the drive element (3) and metering element (4) assembly forms at least one selector element (6),
d) and in which the drive element (3) can perform a translation movement relative to the metering element (4) up to a trip position, at which at least one selector element (6) is engaged with one of the metering stops (8) and
e) in the trip position at least one selector element (6) with the rotation stops (5) prevents the rotation of the metering element (4) in both directions of rotation.

2. Drive and metering module according to the foregoing claim, **characterised in that** the metering element (4) for the adjustment of the product dose can be rotated relative to a drive element (3) to one of a number of metering positions, whereby the drive element is mechanically coupled with the driven element and can perform a translation movement relative to the driven element.

3. Drive and metering module according to one of the foregoing claims, **characterised in that** the product dose depends on the axially different heights of the many metering stops (8).

4. Drive and metering module according to one of the previous claims, **characterised in that** the metering stops (8) are formed respectively from the front face (8) of a slot (9), which extends axially from a front face (7) of the component (3;4) containing the metering stops (8).

5. Drive and metering module according to the previous claim, **characterised in that** the rotation stops (5) are formed by the flanks (5) of the slot (9).

6. Drive and metering module according to one of the previous claims, **characterised in that** the axially aligned metering stops (8) of the drive element (3) and metering element (4) combination are arranged in the form of steps, whereby the flanks (5) of the slots form the rotation stops (5).

7. Drive and metering module according to one of the previous claims, **characterised in that** the metering stops (8) are formed by the metering element (4) and at least one selector element (6) through the driven element (3).

8. Drive and metering module according to one of the previous claims, **characterised in that** at least one selector element (6) is a web or rib extending in the axial direction, which preferably protrudes from the shell of the drive element (3) or of the metering element (4).

9. Drive and metering module according to one of the previous claims, **characterised in that** the metering element (4) is mechanically coupled with a mechanical holder (1), in which the metering element (4) can rotate relative to the mechanical holder (1).

10. Drive and metering module according to one of the previous claims, **characterised in that** the drive element (3) is mechanically coupled with a mechanical holder (1), in which the drive element (3) can perform a translation movement relative to the mechanical holder (1).

11. Drive and metering module according to one of the previous claims, **characterised in that** a delivery device (3-7) comprises a manually operable actuating element (6), whose actuation causes the delivery motion, and that a path length (H_{F} + H_{L}) of a movement of the actuating element (6) on actuation is greater than that of the path length (H_{F}) corresponding to the delivery movement required for the complete discharge of the set product dose.

12. Drive and metering module according to the previous claim, **characterised in that** the delivery device (3-7) has at least one delivery element (3) arranged in a reservoir (2), which carries out the delivery movement and that the actuating element (6) is coupled with the delivery element (3) in such a way, that over a portion (H_{F}) of the path length (H_{F} + H_{L}) of its movement it carries with it the delivery element (3) and traverses the other portion (H_{L}) of the path length (H_{F} + H_{L}) of its movement without the delivery element (3).

13. Drive and metering module for an injection device, comprising
a) a metering element (4),
b) a mechanical holder (1) relative to which the metering element (4) can be rotated,
c) in which at least one latching element (15) formed in the mechanical holder (1) and metering element (4) assembly is provided and
d) in which at least one counter-latching element (16) formed in the mechanical holder (1) and metering element (4) assembly, is provided, in which the latching element (15) can engage and block the rotation of the metering element (4) relative to the mechanical holder (1).

14. Drive and metering module according to the foregoing claim, **characterised in that** the latching element (15) is provided with a means of spring loading in the axial direction.

15. Drive and metering module according to one of the two foregoing claims, **characterised in that** the latching element (15) is formed on the metering element (4).

16. Drive and metering module according to one of the three foregoing claims, **characterised in that** the number of counter-latching elements (16) is equal to or greater than the number of latching elements (15).

17. Drive and metering module according to one of the four foregoing claims, **characterised in that** the metering element (4) and drive element (3) assembly has a number of metering stops (8) arranged around the circumference, in which a number of counter latching elements (16) have the same angular separation (W) from each other as the metering stops (8).

18. Drive and metering module according to one of the five foregoing claims, **characterised in that** a blocking element (17) can hold the latching element (15) in engagement with the counter latching element (16), by which means the rotation of the metering element (4) relative to the mechanical holder (1) is blocked.

19. Drive and metering module according to the foregoing claim, **characterised in that** the blocking element (17) is formed on or formed from an actuating element (10).

20. Drive and metering module according to one of the claims 13 or 19, **characterised in that** at least in a release position a blocking element (17) releases the latching element (15).

21. Drive and metering module according to one of the claims 13 to 20, **characterised in that** a blocking element (17) has an axial length, which is shorter than the path, over which the blocking element (17) can be moved in the case of a set maximum product dose minus twice the axial length of the latching element (15).

22. Drive and metering module according to one of the claims 13 to 21, **characterised in that** the drive and metering module has at least one of the features of claims 1 to 12.

23. Injection device for the injection of a set product dose, comprising a drive and metering module, which corresponds to at least one of the claims 1 to 12 in combination with at least one of the claims 13 to 21.

## Revendications

1. Module d'entraînement et de dosage pour un appareil d'injection, comprenant :
a) un organe de dosage (4) qui peut être déplacé relativement à un organe d'entraînement (3) dans une position de dosage parmi plusieurs afin de régler la dose de produit,
b) l'un parmi l'organe d'entraînement (3) et l'organe de dosage (4) formant plusieurs butées de dosage (8) à des hauteurs différentes axialement et des butées rotatives (5) attribuées aux butées de dosage (8),
c) et l'autre module parmi l'organe d'entraînement (3) et l'organe de dosage (4) formant au moins un élément de sélection (6),
d) et l'organe d'entraînement (3) pouvant être déplacé en translation relativement à l'organe de dosage (4) jusque dans une position de déclenchement, dans laquelle ledit au moins un élément de sélection (6) est en butée contre une des butées de dosage (8) et
e) dans la position de déclenchement ledit au moins un élément de sélection (6) empêche, avec les butées rotatives (5), la rotation de l'organe de dosage (4) dans les deux sens de rotation.

2. Module d'entraînement et de dosage selon la revendication précédente, **caractérisé en ce que** l'organe de dosage (4) peut tourner relativement à un organe d'entraînement (3) dans une position de dosage parmi plusieurs afin de régler la dose de produit, l'organe d'entraînement étant couplé mécaniquement avec l'organe d'entraînement et pouvant être déplacé en translation relativement à l'organe d'entraînement.

3. Module d'entraînement et de dosage selon l'une quelconque des deux revendications précédentes, **caractérisé en ce que** la dose de produit dépend des hauteurs différentes axialement des multiples butées de dosage (8).

4. Module d'entraînement et de dosage selon l'une quelconque des revendications précédentes, **caractérisé en ce que** les butées de dosage (8) sont formées par respectivement une face frontale (8) d'une rainure (9) qui s'étend axialement depuis une face frontale (7) du composant (3 ; 4) contenant les butées de dosage (8).

5. Module d'entraînement et de dosage selon la revendication précédente, **caractérisé en ce que** les butées rotatives (5) sont formées par les flancs (5) de la rainure (9).

6. Module d'entraînement et de dosage selon l'une quelconque des revendications précédentes, **caractérisé en ce qu'**au niveau de l'un parmi l'organe d'entraînement (3) et l'organe de dosage (4), les butées de dosage (8) à indication axiale vers l'autre organe sont disposées en gradins, les flancs (5) des rainures formant les butées rotatives (5).

7. Module d'entraînement et de dosage selon l'une quelconque des revendications précédentes, **caractérisé en ce que** les butées de dosage (8) sont formées par l'organe de dosage (4) et l'élément de sélection (6) au moins au nombre de un est formé par l'organe d'entraînement (3).

8. Module d'entraînement et de dosage selon l'une quelconque des revendications précédentes, **caractérisé en ce que** l'élément de sélection (6) au moins au nombre de un est une came ou une barrette passant dans le sens axial, lesquels dépassent de préférence d'une surface latérale de l'organe d'entraînement (3) ou de l'organe de dosage (4).

9. Module d'entraînement et de dosage selon l'une quelconque des revendications précédentes, **caractérisé en ce que** l'organe de dosage (4) est couplé mécaniquement avec un support de mécanisme (1), l'organe de dosage (4) pouvant tourner relativement au support de mécanisme (1).

10. Module d'entraînement et de dosage selon l'une quelconque des revendications précédentes, **caractérisé en ce que** l'organe d'entraînement (3) est couplé mécaniquement avec un support de mécanisme (1), l'organe d'entraînement (3) pouvant être déplacé en translation relativement au support de mécanisme (1).

11. Module d'entraînement et de dosage selon l'une quelconque des revendications précédentes, **caractérisé en ce qu'**un convoyeur (3-7) comprend un élément d'actionnement (6) pouvant être actionné à la main, dont l'actionnement provoque le mouvement de transport, et **en ce qu'**une longueur de parcours (H_{F}+H_{L}) d'un mouvement exécutable par l'élément d'actionnement (6) lors de l'actionnement est supérieure à la longueur de parcours (H_{F}) du mouvement de transport correspondant au déversement complet de la dose de produit réglée.

12. Module d'entraînement et de dosage selon la revendication précédente, **caractérisé en ce que** le convoyeur (3-7) comporte au moins un organe de transport (3) disposé dans un réservoir (2), ledit organe exécutant le mouvement de transport, et **en ce que** l'élément d'actionnement (6) est couplé de telle façon avec l'organe de transport qu'il entraîne l'organe de transport (3) sur une portion (H_{F}) de la longueur de parcours (H_{F}+H_{L}) de son mouvement, et parcourt l'autre portion (H_{L}) de la longueur de parcours de son mouvement (H_{F}+H_{L}) sans l'organe de transport (3).

13. Module d'entraînement et de dosage pour un appareil d'injection, comprenant :
a) un organe de dosage (4),
b) un support de mécanisme (1) pouvant tourner relativement à l'organe de dosage (4),
c) au moins un élément d'arrêt (15) étant formé au niveau de l'un parmi le support de mécanisme (1) et l'organe de dosage (4) et
d) au moins un contre-élément d'arrêt (16) étant formé au niveau de l'autre parmi le support de mécanisme (1) et l'organe de dosage (4), l'élément d'arrêt (15) pouvant engrener dans ledit contre-élément et empêcher la rotation de l'organe de dosage (4) relativement au support de mécanisme (1).

14. Module d'entraînement et de dosage selon la revendication précédente, **caractérisé en ce que** l'élément d'arrêt (15) est disposé de façon élastique dans le sens radial.

15. Module d'entraînement et de dosage selon l'une quelconque des deux revendications précédentes, **caractérisé en ce que** l'élément d'arrêt (15) est formé sur l'organe de dosage (4).

16. Module d'entraînement et de dosage selon l'une quelconque des trois revendications précédentes, **caractérisé en ce que** le nombre de contre-éléments d'arrêt (16) est supérieur ou égal au nombre d'éléments d'arrêt (15).

17. Module d'entraînement et de dosage selon l'une quelconque des quatre revendications précédentes, **caractérisé en ce que** l'un parmi l'organe de dosage (4) et l'organe d'entraînement (3) comporte plusieurs butées de dosage (8) disposées sur le pourtour, plusieurs contre-éléments d'arrêt (16) ayant, les uns par rapport aux autres, la même division angulaire (W) que les butées de dosage (8).

18. Module d'entraînement et de dosage selon l'une quelconque des cinq revendications précédentes, **caractérisé en ce qu'**un élément de blocage (17) peut maintenir l'élément d'arrêt (15) dans un engrènement d'arrêt avec le contre-élément d'arrêt (16), ce qui empêche la rotation de l'organe de dosage (4) relativement au support de mécanisme (1).

19. Module d'entraînement et de dosage selon la revendication précédente, **caractérisé en ce que** l'élément de blocage (17) est formé sur un ou par un élément d'actionnement (10).

20. Module d'entraînement et de dosage selon l'une quelconque des revendications 13 ou 19, **caractérisé en ce que** au moins dans une position de libération un élément de blocage (17) libère l'élément d'arrêt (15).

21. Module d'entraînement et de dosage selon l'une quelconque des revendications 13 à 20, **caractérisé en ce qu'**un élément de blocage (17) comporte une longueur axiale inférieure au parcours que l'élément de blocage (17) peut parcourir en translation dans le cas d'une dose de produit réglée au maximum, moins le double de la longueur axiale de l'élément d'arrêt (15).

22. Module d'entraînement et de dosage selon l'une des revendications 13 à 21, **caractérisé en ce que** le module d'entraînement et de dosage comporte au moins une des caractéristiques des revendications 1 à 12.

23. Appareil d'injection pour injecter une dose de produit réglable, comprenant un module d'entraînement et de dosage, qui correspond au moins à une des revendications 1 à 12 en combinaison avec au moins une des revendications 13 à 21.
